# EUROPÄISCHE PATENTSCHRIFT

(11) **EP 2 637 932 B1**
(45) Veröffentlichungstag und Bekanntmachung des Hinweises auf die Patenterteilung: **16.07.2014**
(21) Anmeldenummer: 11740843.5
(22) Anmeldetag: 27.07.2011
(51) Int. Cl.: B65B 3/00, A61M 1/02, F16K 7/04, F16K 15/04

(54) **BEHÄLTERANORDNUNG UND VERFAHREN ZUM FÜLLEN VON FLEXIBLEN EINWEGBEUTELN**
CONTAINER ARRANGEMENT AND METHOD FOR FILLING FLEXIBLE DISPOSABLE BAGS
ARRANGEMENT DE RECIPIENT ET PROCEDE DE REMPLISSAGE DE SACHETS A USAGE UNIQUE SOUPLES

(30) Priorität: 10.11.2010 DE 102010060469
(43) Veröffentlichungstag der Anmeldung: 18.09.2013
(73) Patentinhaber: Sartorius Lab Instruments GmbH & Co. KG, 37075 Göttingen (DE)
(72) Erfinder: MÜLLER, Michael, 37079 Göttingen (DE)
(74) Vertreter: Schneider, Peter Christian
(86) Internationale Anmeldenummer: PCT/EP2011/003786
(87) Internationale Veröffentlichungsnummer: WO 2012/062383

(56) Entgegenhaltungen:
- EP-A2- 1 693 077
- EP-B1- 1 525 138
- WO-A1-93/09863
- US-A- 3 430 648
- US-A1- 2005 109 795
- US-A1- 2010 236 340

## Beschreibung

### Gebiet der Erfindung

Die Erfindung bezieht sich auf eine Behälteranordnung, umfassend eine Mehrzahl von flexiblen Einwegbeuteln, welche jeweils ein Einlasselement aufweisen, an das ein flexibler Verbindungsschlauch angeschlossen ist, wobei jeder Verbindungsschlauch von einer flexiblen, gemeinsamen Hauptleitung, die eingangsseitig einen gemeinsamen Einlassabschnitt aufweist, abzweigt.

Die Erfindung bezieht sich weiter auf ein Verfahren zum Befüllen der flexiblen Einwegbeutel einer solchen Behälteranordnung.

### Stand der Technik

Aus der EP 1 525 138 B1 ist eine gattungsgemäße Behälteranordnung bekannt. Derartige Behälteranordnungen folgen dem allgemeinen Trend in der Medizintechnik und der Biotechnologie weg von wiederverwendbaren Behältern zu sogenannten Einwegbehältern, die für die einmalige Benutzung und anschließende Entsorgung ausgelegt sind. Die Behälter sind als flexible Kunststoffbeutel mit jeweils wenigstens einem Einlasselement ausgebildet. Jeder Beutel ist über einen ihm zugeordneten flexiblen Verbindungsschlauch an eine Hauptleitung angeschlossen, die regelmäßig ebenfalls als flexibler Schlauch ausgebildet ist. Eingangsseitig weist die Hauptleitung einen Einlassabschnitt auf, der beispielsweise an eine Pumpe angeschlossen werden kann. Es ist bekannt, solche Behälteranordnungen als vorkonfektionierte, sterile Einheiten zu handeln. Ihre Verwendung erfolgt in einer Abfüllanlage, die eine Mehrzahl von Klemmventilen aufweist, wobei jeder Verbindungsschlauch in ein zugeordnetes Klemmventil eingelegt wird. Der Einlassabschnitt der Hauptleitung wird an eine mit einem Vorratsbehälter verbundene Pumpe angeschlossen. Es ist ebenso bekannt, den Einlassabschnitt der Hauptleitung unmittelbar an den Vorratsbehälter oder ein diesem zugeordnetes Absperrventil anzuschließen, wobei der Vorratsbehälter entweder selbst druckbeaufschlagt ist oder höher positioniert ist als die als Zielbehälter dienenden Einwegbeutel der Behälteranordnung. Durch abwechselndes, einzelnes Öffnen der Klemmventile können die Zielbeutel nacheinander aus dem Vorratsbehälter befüllt werden, wobei ihr Füllstand beispielsweise gravimetrisch überwacht werden kann. Nachteilig bei dem bekannten System ist das Fehlen einer Entgasungsmöglichkeit zur Abführung von Restgas in dem Schlauchsystem vor der Befüllung der Einwegbeutel. Dies führt dazu, dass das Restgas wenigstens in einen der Beutel abgefüllt wird, was in vielen Fällen unerwünscht ist.

Aus der EP 1 236 644 ist ein System zur Befüllung eines Zielbeutels aus einer Mehrzahl von Vorratsbeuteln bekannt. Alle Beutel sind über ihnen jeweils zugeordnete Verbindungsschläuche an ein gemeinsames Verteilerstück angeschlossen. Das Verteilerstück weist einen gemeinsamen Zentralraum auf, der eine Mehrzahl von Ein-/Ausgängen hat, welche jeweils von einem Schaltventil verschlossen sind. Jeder Verbindungsschlauch ist an einen derartigen Ein-/Ausgang angeschlossen. An einem weiteren Ein-/Ausgang ist eine Kolbenpumpe in Form einer typischen Injektionsspritze angeschlossen. An einem weiteren Ein-/Ausgang ist ein mit einem Membranfilter versehener Gasauslassstutzen angeschlossen. Zum Befüllen wird der Kolben der Kolbenpumpe phasenweise zurückgezogen und vorgeschoben, wobei der Volumenfluss von den Vorratsbeuteln zum Zielbeutel durch entsprechende Steuerung der Schaltventile gesteuert wird. Restgas aus dem Schlauchsystem, welches sich im Zentralraum des Verteilerstücks ansammelt, kann durch entsprechende Steuerung des dem Gasauslassstutzen zugeordneten Schaltventils abgeführt werden. Nachteilig ist dabei die technisch aufwendige und daher teure Ausgestaltung des Verteilerstücks, welches die Schaltventile enthält. Einerseits stellt es das zentrale Element des Schlauchsystems dar, an welches alle Verbindungsschläuche angeschlossen werden müssen. Andererseits kann es aus Kostengründen nicht als Einwegelement ausgestaltet sein. Vielmehr handelt es sich um ein wiederverwendbares Element, das nach jeder Benutzung aufwendig gereinigt und sterilisiert werden muss. Mit anderen Worten müssen die typischerweise steril und vorkonfektionierten Verbindungsschlauch/Beutel-Anordnungen vor jedem Befüllvorgang an ein hygienisch kritisches Zentralelement angekoppelt werden, wobei der Kopplungsvorgang selbst ebenfalls hygienisch kritisch ist.

### Aufgabenstellung

Es ist die Aufgabe der vorliegenden Erfindung, eine gattungsgemäße Behälteranordnung mit einer kostengünstigen Entgasungsmöglichkeit zu versehen, ohne dabei hygienische Nachteile in Kauf nehmen zu müssen.

### Darlegung der Erfindung

Diese Aufgabe wird in Verbindung mit den Merkmalen des Oberbegriffs von Anspruch 1 dadurch gelöst, dass die Hauptleitung ausgangsseitig einen gemeinsamen Gasauslassabschnitt aufweist, in dem ein entgegen der Gasauslassrichtung schließendes Rückschlagventil und ein gasdurchlässiger, flüssigkeitsdichter Sterilfilter angeordnet sind.

Erfindungsgemäß wird die Hauptleitung des Schlauchsystems mit einem ausgangsseitigen Gasauslassabschnitt versehen. Das bedeutet, dass die Hauptleitung im Wesentlichen aus drei Abschnitten besteht, nämlich einem Einlassabschnitt, einem sich daran anschließenden Mittelabschnitt, von dem die Verbindungsschläuche zu den einzelnen Einwegbeuteln abzweigen und einen sich daran anschließenden Gasauslassabschnitt. Diese Anordnung erlaubt es, bei einem Befüllvorgang, bei dem alle Verbindungsschläuche in ihnen jeweils zugeordnete Klemmventile eingelegt und mittels dieser verschlossen sind, zunächst Flüssigkeit aus einem mit dem Einlassabschnitt der Hauptleitung verbundenen Vorratsbehälter in die Hauptleitung und die davon abzweigenden Verbindungsschläuche (dort bis zu den jeweils sperrenden Klemmventilen) einzuleiten und dabei das im Schlauchsystem befindliche Restgas entlang der Hauptleitung in den Gasauslassabschnitt und durch den gasdurchlässigen Sterilfilter nach außen zu drücken. Der Sterilfilter ist erforderlich, um einen Keimeintrag von außen in das nunmehr erfindungsgemäß offene Schlauchsystem zu verhindern.

Diese Maßnahmen allein würden jedoch zur Lösung der Aufgabe noch nicht ausreichen. Öffnete man nämlich anschließend zur Befüllung eines ersten Einwegbeutels das diesem zugeordnete Klemmventil, bestünde die Gefahr, dass die Vorflutflüssigkeit, d.h. diejenige Flüssigkeit, die zum Austreiben des Restgases in die Hauptleitung gefördert wurde, in den geöffneten Einwegbeutel hingepresst würde. Diese Gefahr besteht in besonderem Maße in den Fällen, in denen die zu befüllenden Einwegbeutel übereinander z.B. in einem Stapelregal gestapelt sind. Der Druck der Flüssigkeitssäule über dem Abzweig des gerade zu befüllenden Einwegbeutels würde die Flüssigkeit aus der Hauptleitung und dem Verbindungsschlauch in den Einwegbeutel drücken. Dies würde insbesondere bei einer gravimetrischen Füllstandsüberwachung zu Messfehlern führen. Bei der gravimetrischen Füllstandsüberwachung ist nämlich das gesamte System aus Einwegbehältern, Verbindungsschläuchen und Hauptleitung auf einer gemeinsamen Wägeplattform angeordnet, sodass gravimetrisch nicht unterschieden werden kann, ob sich ein Flüssigkeitsanteil in der Hauptleitung oder in einem Einwegbeutel befindet.

Weiter erfindungsgemäß ist daher vorgesehen, dass in dem Gasauslassabschnitt zusätzlich ein Rückschlagventil angeordnet ist. Dieses ist so orientiert, dass es einen Fluidstrom in Gasauslassrichtung zulässt und entgegen der Gasauslassrichtung sperrt. Dadurch wird ein Rückstrom der Vorflutflüssigkeit in einen Einwegbeutel beim Öffnen von dessen zugeordnetem Klemmventil verhindert. Ein Absacken der Flüssigkeitssäule in der Hauptleitung würde nämlich zu einem Unterdruck am Rückschlagventil führen, der der Schwerkraft entgegenwirkt und die Flüssigkeitssäule in der Hauptleitung hält. Ein beispielsweise pumpengetriebener Fluidstrom von dem Vorratsbehälter in den Einwegbeutel bliebe von dem Unterdruck am Rückschlagventil hingegen weitestgehend unbeeinflusst.

Bevorzugt sind das Rückschlagventil und der Sterilfilter in einem gemeinsamen Gehäuse angeordnet, d.h. als ein gemeinsames Bauteil oder Modul ausgebildet. Diese Maßnahme trägt zur Montagesicherheit und Bauraumoptimierung bei.

Die Anordnung des Rückschlagventils relativ zum Sterilfilter kann auf unterschiedliche Arten erfolgen. Bei einer ersten Ausführungsform ist vorgesehen, dass das Rückschlagventil in Gasauslassrichtung hinter dem Sterilfilter angeordnet und als Gasventil ausgebildet ist. Das auszutreibende Gas passiert also somit zunächst den Sterilfilter und danach das Rückschlagventil. Bei dieser Anordnung kommt das Rückschlagventil zu keinem Zeitpunkt mit der Vorflutflüssigkeit in Kontakt, da diese den flüssigkeitsdichten Sterilfilter nicht passieren kann. Entsprechend ist das Rückschlagventil als Gasventil auszugestalten, d.h. als ein Ventil, welches im geschlossenen Zustand gasdicht ist.

Wie erläutert, markiert der Sterilfilter die maximale Füllhöhe des Gasauslassabschnitts bzw. der Hauptleitung. Das bedeutet, dass sich auch bei maximaler Vorflutung zwischen dem Sterilfilter und dem Rückschlagventil ein Gaskissen bildet. Sackt beim Öffnen eines Klemmventils eines Einwegbeutels die Flüssigkeitssäule in der Hauptleitung schwerkraftbedingt ab, schließt zwar das Rückschlagventil; aufgrund der Kompressibilität von Gasen expandiert jedoch das Gaskissen wenigstens geringfügig, sodass die Vorflutflüssigkeit nicht vollständig in der Hauptleitung gehalten werden kann, sondern ein - wenn auch geringer - Anteil in den Einwegbeutel fließt. Der hieraus resultierende Füllmengenfehler kann, da seine Größe in Tests ohne weiteres bestimmbar ist, durch eine entsprechende Minderbefüllung des Einwegbeutels kompensiert werden. Weiter kann der Fehler reduziert werden, indem der Abstand zwischen dem Sterilfilter und dem Rückschlagventil und somit das Gaskissen minimiert wird.

Nahezu vollständig vermieden wird ein derartiger Befüllungsfehler bei einer alternativen Ausführungsform, bei der vorgesehen ist, dass das Rückschlagventil in Gasauslassrichtung vor dem Sterilfilter angeordnet und als Flüssigkeitsventil ausgebildet ist. Bei maximaler Vorflutung, d.h. bei Vorflutung bis zum Sterilfilter, wird das Rückschlagventil von der Vorflutflüssigkeit durchströmt. Folglich muss das Rückschlagventil als Flüssigkeitsventil ausgebildet sein, d.h. als ein Ventil, welches in der Lage ist, flüssigkeitsdicht zu sperren.

Bei dieser Ausführungsform muss bei hinreichend hoher Vorflutung kein Gaskissen vor dem Rückschlagventil entstehen. Aufgrund der äußerst geringen Kompressibilität von Flüssigkeiten sackt die Flüssigkeitssäule in der Hauptleitung beim Öffnen eines einem Einwegbeutel zugeordneten Klemmventils praktisch nicht ab. Der resultierende Befüllungsfehler ist vernachlässigbar.

Problematisch bei dieser Ausführungsform kann der Kontakt zwischen den inneren Elementen des Rückschlagventils und der Vorflutflüssigkeit sein. Typische Rückschlagventile sind aus metallischen Werkstoffen gebildet, die mit empfindlichen biotechnologischen Flüssigkeiten unter Umständen in unerwünschter Weise reagieren können. Um dies zu verhindern, ist bei einer Weiterbildung der Erfindung vorgesehen, dass alle inneren Elemente des Rückschlagventils aus Kunststoff gefertigt sind. Als geeigneter Kunststoff wird bevorzugt ein inertes Material verwendet, welches mit der bestimmungsgemäßen Flüssigkeit nicht reagiert. Beispielsweise kann eine steifere Variante desselben Kunststoffs verwendet werden, aus dem auch die Verbindungsschläuche und die Hauptleitung gebildet sind.

Die bevorzugte Vertriebsform der erfindungsgemäßen Behälteranordnung ist als vorkonfektionierte Einheit. Zur Platzersparnis und leichteren Lagerfähigkeit ist bevorzugt vorgesehen, dass die flexiblen Einwegbeutel evakuiert sind. Sie nehmen daher kaum mehr Raum ein als eine Folie und können leicht gestapelt werden.

Die Vorkonfektionierung der Verbindungsschläuche und der Hauptleitung kann auf unterschiedliche Weisen erfolgen. Grundsätzlich ist es möglich, das gesamte Schlauchsystem und ggfs. auch die Einwegbeutel einstückig herzustellen. Dies ist jedoch technisch sehr aufwendig und im Hinblick auf unterschiedliche Beutelzahlen, Beutelgrößen, Leitungslängen, Leitungsdurchschnitte etc. wenig flexibel. Die Vorkonfektionierung erfolgt daher bevorzugt durch ein Verbinden einzelner Einwegbeutel und einzelner flexibler Schlauchabschnitte mittels Schlauchverbindern, die z.B. als zu ihren Enden hin konisch angespitzte Röhrchen oder Verbindungskreuze mit zu den Enden hin konisch angespitzten Armen, vorzugsweise mit drei Armen, ausgebildet sein können. Die Hauptleitung besteht bei einer solchen Ausgestaltung aus einer Mehrzahl von Schlauchabschnitten, die über dreiarmige Schlauchverbinder miteinander verbunden sind, wobei der jeweils dritte Arm der Schlauchverbinder einen Abzweig zu einem Verbindungsschlauch darstellt, der anderenends mittels eines zweiarmigen Schlauchverbinders mit der Eingangsöffnung des Einwegbeutels verbunden ist. Evtl. können die Schlauchverbindungen noch mit Sicherheitsbändern oder-spangen gesichert sein.

Bei einer Weiterbildung der bevorzugten Vertriebsform der erfindungsgemäßen Behälteranordnung ist weiter vorgesehen, dass diese von einer flexiblen, gasdichten Hülle umgeben ist und als Gesamtheit sterilisiert wurde. Dies bedeutet mit anderen Worten, dass das Systems aus Schläuchen und Einwegbeuteln nach seiner Konfektionierung in eine keimdichte Hülle verpackt, z.B. in einen Kunststoffbeutel eingeschweißt wird. Die Sterilisierung des Gesamtpaketes erfolgt nach dieser Form der Verpackung beispielsweise durch Erhitzen oder durch Bestrahlung, beispielsweise durch Gammastrahlung. Diese Vorgehensweise erlaubt es, die Vorkonfektionierung und Verpackung der Behälteranordnung unter nicht-sterilen Bedingungen durchzuführen, was die entsprechenden Kosten deutlich reduziert. Gleichwohl erhält der Benutzer eine nach seinen Wünschen vorkonfektionierte und sterile Einheit, die er lediglich in seine Befüllanlage einlegen und mit dem oder den Vorratsbehältern, aus denen die Einwegbeutel befüllt werden sollen, verbinden muss. Es verbleibt somit lediglich ein einziger hygienisch kritischer Schritt im Verantwortungsbereich des Benutzers, nämlich der Anschluss der erfindungsgemäßen Behälteranordnung an den oder die Vorratsbehälter.

Bei dem bevorzugten Verfahren zum Befüllen der flexiblen Einwegbeutel einer erfindungsgemäßen Behälteranordnung werden die Einwegbeutel in ein auf einer elektronischen Waage positioniertes Trägergestell eingesetzt, jeder Verbindungsschlauch wird in ein dem jeweils angeschlossenen Einwegbeutel zugeordnetes, steuerbares Ziel-Klemmventil eingelegt und der Einlassabschnitt der Hauptleitung wird mit einem Vorratsbehälter verbunden und in ein steuerbares Vorrats-Klemmventil eingelegt, wobei alle Klemmventile zunächst geschlossen sind. Danach werden folgende Schritte durchgeführt:
a) Öffnen des Vorrats-Klemmventils,
b) Entweichenlassen von in der Hauptleitung enthaltenem Gas durch den Gasauslassabschnitt,
c) Tarieren der elektronischen Waage,
d) Öffnen eines ersten Ziel-Klemmventils,
e) Befüllen des zugeordneten Einwegbeutels unter Überwachung einer Gewichtsanzeige der elektronischen Waage bis zum Erreichen eines vorgegebenen Füllgewichts,
f) Schließen des ersten Ziel-Klemmventils,
g) jeweils Wiederholen der Schritte c-f für alle weiteren Ziel-Klemmventile und zugeordneten Einweg-Beutel.

Man beachte, dass beim Tarieren der elektronischen Waage die Vorflutflüssigkeit in der Hauptleitung mitberücksichtigt wird. Durch die erfindungsgemäße Ausgestaltung des Gasauslassabschnitts führt das Öffnen des ersten Ziel-Klemmventils jedoch nicht, wie eigentlich zu erwarten, zu einem Einströmen der Vorflutflüssigkeit in den zugeordneten ersten Einwegbeutel. Vielmehr kann der erste Einwegbeutel allein aus dem Vorratsbehälter, nämlich über den Einlassabschnitt und den dem ersten Einwegbeutel zugeordneten ersten Verbindungsschlauch befüllt werden. Lediglich die im Einlassabschnitt und im ersten Verbindungsschlauch enthaltene Vorflutflüssigkeit wird dabei in den ersten Einwegbeutel gedrückt. Dies führt jedoch nicht zu einem Befüllfehler. Wird nämlich der Befüllvorgang des ersten Einwegbeutels durch Schließen des ersten Ziel-Klemmventils in dem Moment gestoppt, in dem die Anzeige der elektronischen Waage das vorgegebene Füllgewicht anzeigt, ist die von dem Einlassabschnitt und dem ersten Verbindungsschlauch in den ersten Einwegbeutel gedrückte Vorflutflüssigkeit durch die zuletzt geförderte Vorratsflüssigkeit ersetzt und trägt zu dem auf der elektronische Waage lastenden Gewicht bei. Das angezeigte Füllgewicht entspricht somit exakt dem Gewicht der in den Beutel eingefüllten Flüssigkeit. Entsprechendes gilt für die nachfolgenden Befüllungen der übrigen Einwegbeutel, die *mutatis mutandis* nach denselben Verfahrensschritten vollzogen werden.

Man beachte, dass der Begriff des Tarierens der elektronischen Waage hier weit zu verstehen ist und sowohl die tatsächliche Null-Stellung bei gegebener Grundbelastung der Wägeplattform umfasst als auch ein rechnerisches Tarieren, bei dem ein aktueller, von Null verschiedener Anzeigewert gespeichert wird, der zur Ermittlung des Füllgewichtes jeweils von dem von der Anzeige angezeigten Wert abzuziehen ist.

Der Begriff der Anzeige ist hier ebenfalls weit zu verstehen und ist insbesondere nicht auf eine visuelle Klartextanzeige beschränkt. Vielmehr sei auch die Ausgabe elektronischer Daten zur Weiterverwendung in einer elektronischen Steuereinheit ohne Zwischenschaltung menschlicher Arbeitsschritte umfasst.

Überhaupt ist eine vollautomatische Durchführung des Verfahrens die bevorzugte Ausführungsform, wobei das Vorsehen und die Einrichtung einer entsprechenden Steuereinrichtung sowie ihrer wirksamen Verbindungen zu den aktiven Elementen für den Fachmann im Lichte der hiesigen Offenbarung ohne weiteres durchführbar ist.

Günstigerweise wird die erfindungsgemäße Behälteranordnung in dem Trägergestell so positioniert, dass der Gasauslassabschnitt nahe oder an der höchsten Stelle der Behälteranordnung positioniert ist. Bei dieser Positionierung kann die Erfindung ihre Wirkung am vorteilhaftesten entfalten.

Weitere Merkmale und Vorteile der Erfindung ergeben sich aus der nachfolgenden, speziellen Beschreibung und der Zeichnung.

### Kurzbeschreibung der Zeichnungen

Es zeigt:

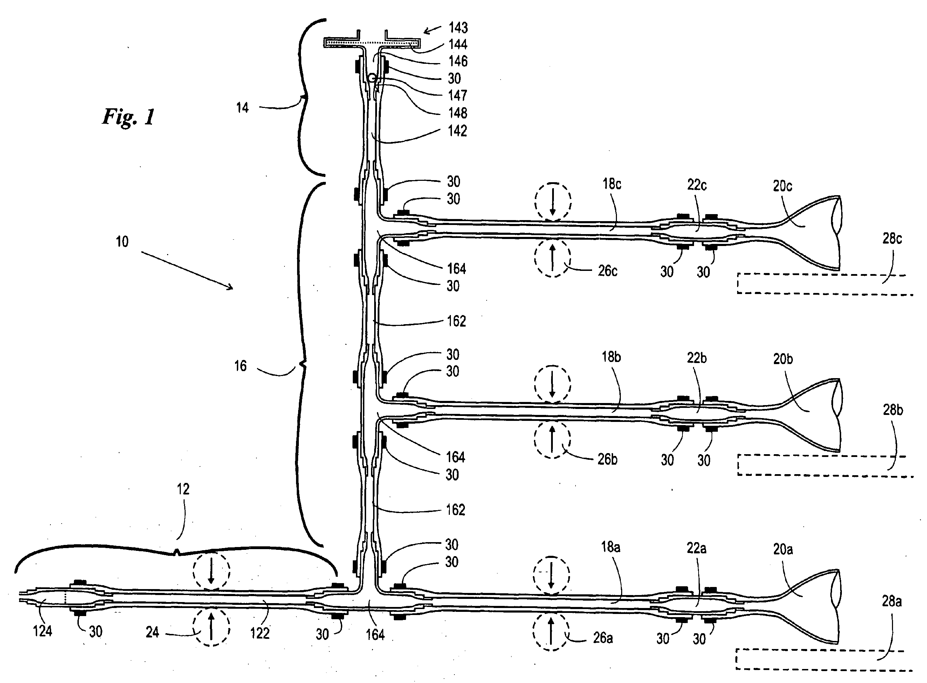

- Figur 1:: eine schematische Querschnittsdarstellung einer erfindungsgemäßen Behälteranordnung.

### Ausführliche Beschreibung bevorzugter Ausführungsformen

Figur 1 zeigt eine schematische Darstellung einer erfindungsgemäßen Behälteranordnung 10. Die Behälteranordnung 10 weist einen Einlassabschnitt 12 und einen Gasauslassabschnitt 14 auf. Dazwischen erstreckt sich eine Hauptleitung 16, von der Verbindungsschläuche 18a, b, c zu jeweils einem Einwegbeutel 20a, b, c abzweigen. Die Anzahl der Abzweigungen, d.h. insbesondere die Anzahl der Verbindungsschläuche 18a, b, c und der Einwegbeutel 20a, b, c ist nicht auf die hier dargestellte Anzahl drei beschränkt. Vielmehr kann jede beliebige Mehrzahl von Abzweigungen realisiert werden. Im Fachjargon wird dabei allgemein als "Manyfold" gesprochen.

Bei der dargestellten Ausführungsform besteht der Einlassabschnitt 12 im Wesentlichen aus zwei Komponenten, nämlich einem flexiblen Einlassschlauch 122 und einem eingangsseitig mit diesem verbundenem Sterilverbinder 124. Der Sterilverbinder 124 dient der Kopplung des Einlassschlauchs 122 mit einem oder mehreren Vorratsbehältern, aus denen eine Flüssigkeit in die Einwegbeutel 20a, b, c abgefüllt werden soll. Die Kopplung kann unmittelbar oder mittelbar beispielsweise über eine Pumpeneinrichtung erfolgen. Die spezielle Ausgestaltung des Sterilverbinders 124 ist für die vorliegende Erfindung ohne Bedeutung; dem Fachmann sind verschiedene Arten von Sterilverbindern 124 bekannt. Für die vorliegende Erfindung ist es nicht zwingend erforderlich, wenngleich durchaus vorteilhaft, wenn der Einlassabschnitt 12 einen Sterilverbinder 124 umfasst. Einfachere Ausführungsformen der Erfindung können auch einen herkömmlichen Schlauchverbinder, einen einfachen Schlauchstopfen oder auch nur den unverschlossenen Einlassschlauch 122 aufweisen.

Der Einlassabschnitt 12 ist flüssigkeitsleitend mit der Hauptleitung 16 verbunden. Diese setzt sich bei der gezeigten Ausführungsform aus einer Mehrzahl von dreiarmigen Schlauchverbindern 164 und zwischen diesen angeordneten Hauptleitungsschläuchen 162 zusammen. Bei der dargestellten Ausführungsform verläuft die Hauptleitung 16 im Wesentlichen gerade. Dies ist für die vorliegende Erfindung jedoch nicht von Bedeutung. Aufgabe der Hauptleitung 16 ist es vielmehr, eine durchgehende Verbindung zwischen dem eingangsseitig an sie angeschlossenen Einlassabschnitt 12 und dem ausgangsseitig an sie angeschlossenen Gasauslassabschnitt 14 zu bilden. Eine weitere Aufgabe der Hauptleitung 16 ist es, Abzweigstellen zum Anschluss der Verbindungsschläuche 18a, b, c zur Verfügung zu stellen. Die dargestellte Ausführungsform, bei der sich dreiarmige Schlauchverbinder 164 und Hauptleitungsschläuche 162 abwechseln, ist im Hinblick auf einen widerstandsarmen Flüssigkeitsstrom besonders vorteilhaft. Denkbar sind jedoch auch Ausführungsform mit mehr als dreiarmigen Schlauchverbindern oder stoffschlüssig realisierte Abzweigungen.

An jeder Abzweigung, die bei der gezeigten Ausführungsform durch je einen dreiarmigen Schlauchverbinder 164 realisiert ist, zweigt ein Verbindungsschlauch 18a, b, c ab. Jeder Verbindungsschlauch 18a, b, c ist über einen zweiarmigen Schlauchverbinder 22a, b, c mit dem jeweils zugeordneten Einwegbeutel 20a, b, c, der in Figur 1 nur ausschnittsweise dargestellt ist, verbunden. Anstelle der Kopplung mittels Schlauchverbinder 22a, b, c ist auch hier eine stoffschlüssige Kopplung möglich.

Ausgangsseitig schließt sich an die Hauptleitung 16 ein Gasauslassabschnitt 14 an. Dieser umfasst einen Auslassschlauch 142, der ausgangsseitig mit einem Abschlussmodul 143 verbunden ist. Das Abschlussmodul 143 umfasst im Wesentlichen zwei Funktionselemente, nämlich einen Sterilfilter 144 und ein diesem stromaufwärts vorgelagertes Rückschlagventil 146. Bei der dargestellten Ausführungsform umfasst das Rückschlagventil 146 einen kugelförmigen Ventilkörper 147, der im Fall eines Unterdrucks in der Hauptleitung 16 dichtend an einem Ventilsitz 148 anliegt. Im Fall eines Überdrucks in der Hauptleitung 16 kann sich der Ventilkörper 147 hingegen stromabwärts verlagern und den Ventildurchlass freigeben. Alternativ könnte das Rückschlagventil 146 beispielsweise auch als Membranventil ausgebildet sein. Weiter ist es nicht zwingend erforderlich, dass, wie in Figur 1 gezeigt, das Rückschlagventil 146 stromaufwärts des Sterilfilters 144 angeordnet ist. Vielmehr ist eine umgekehrte Anordnung ebenso möglich.

Bei der in Figur 1 gezeigten Ausführungsform sind sämtliche Schlauchverbindungen mit Schlauchschellen 30 gesichert. Die Notwendigkeit dieser Sicherheitsmaßnahme hat der Fachmann jeweils im Einzelfall anhand der Benutzervorgaben und der Material- und Montagekosten abzuwägen. Für die Erfindung an sich sind die Schlauchschellen 30 nicht von zwingender Notwendigkeit.

In Figur 1 gestrichelt dargestellt sind Elemente einer Abfüllvorrichtung, die bevorzugt zur Befüllung der Einwegbeutel 20a, b, c. eingesetzt wird. Dargestellt sind lediglich diejenigen Elemente, die unmittelbar mit der erfindungsgemäßen Behälteranordnung wechselwirken. Es sind dies ein Vorrats-Klemmventil 24, in welches der Vorratsschlauch 122 eingelegt wird, Ziel-Klemmventile 26a, b, c, in welche jeweils ein Verbindungsschlauch 18a, b, c eingelegt wird, sowie Lagerböden 28a, b, c eines Stapelregals, auf denen die Einwegbeutel 20a, b, c gelagert sind. Weitere Elemente der Befüllvorrichtung, wie insbesondere eine elektronische Waage, auf deren Wägeplattform das Stapelregal angeordnet ist, eine Steuereinheit, welche die Waage und die Klemmventile 24, 26a, b, c ansteuert und Wägewerte der elektronischen Waage erfasst, sowie ein Anschluss zum Vorratsbehälter, ggf. einschließlich einer Pumpe oder der Vorratsbehälter selbst sind in Figur 1 nicht dargestellt. Dem Fachmann sind derartige Befüllvorrichtungen jedoch aus dem einleitend zitierten Stand der Technik grundsätzlich bekannt. Die bevorzugte Verwendung der erfindungsgemäßen Behälteranordnung soll nachfolgend beschrieben werden. Die gesamte Behälteranordnung wird, ggf. unter Zuhilfenahme eines Stapelregals, eines Hängegestells oder einer ähnlichen Vorrichtung, auf der Wägeplattform einer elektronischen Waage positioniert. Der Sterilverbinder 124 des Einlassabschnitts 12 ist zum Beispiel über eine Pumpe mit einem Vorratsbehälter, der eine abzufüllende Flüssigkeit enthält, verbunden. Zu Verfahrensbeginn sind sämtliche Klemmventile 24, 26a, b, c geschlossen, d.h. der Einlassschlauch 122 sowie die Verbindungsschläuche 18a, b, c sind zwischen den Klemmbacken der Ventile 24, 26a, b, c gequetscht, sodass ihr Lumen geschlossen ist. Zu Verfahrensbeginn wird das Vorrats-Klemmventil 24 geöffnet, sodass das Leitungssystem vorgeflutet wird. Beim Vorfluten können sich die Hauptleitung 16 und die davon abzweigenden Verbindungsschläuche 18a, b, c bis zu den jeweiligen Ziel-Klemmventilen 26a, b, c füllen. In dem Schlauchsystem enthaltendes Restgas wird dabei durch die Hauptleitung 16 in den Gasauslassabschnitt 16 gedrückt, wo es das in Gasauslassrichtung öffnende Rückschlagventil 146 passiert und durch den Sterilfilter 144 abgeblasen wird. Weiteres Vorfluten führt zu einem Anstieg der Vorflutflüssigkeit in den Auslassschlauch 142 und weiter in das Abschlussmodul 143. Das Rückschlagventil 146 ist dabei für die Vorflutflüssigkeit passierbar, nicht jedoch der flüssigkeitsdichte Sterilfilter 144. Ein Ende des Vorflutschrittes kann auf unterschiedliche Weise eingeleitet werden. Entweder bestimmt ein in Figur 1 nicht dargestellter Sensor einen vorgegebenen Maximalfüllstand. Alternativ kann ein Druckanstieg in der Hauptleitung 16, dem Einlassabschnitt 12 oder einem vorgeschalteten Leitungsabschnitt bestimmt werden. Der Druckanstieg würde entstehen, wenn eine Pumpe die Vorflutflüssigkeit gegen den sperrenden Sterilfilter 44 drückt. Der maximale Füllstand der Vorflutflüssigkeit wird bevorzugt so eingestellt, dass das Rückschlagventil 146 geflutet, der Sterilfilter 144 jedoch nicht benetzt ist. Es ist jedoch auch möglich, den maximalen Vorflutfüllstand unterhalb des Rückschlagventils 146 anzusetzen. Bei Ausführungsformen, bei denen sich das Rückschlagventil 146 stromabwärts des Sterilfilter 144 befindet, stellt offensichtlich die Position des Sterilfilters 144 die maximal mögliche Füllhöhe dar; das Rückschlagventil 146 ist in diesen Fällen nicht flutbar.

Zum Abschluss des Vorflutschrittes wird die elektronische Waage tariert. D.h. bei nachfolgenden Wägeprozessen wird das Gewicht der Vorflutflüssigkeit nicht berücksichtigt. Mit anderen Worten wird bei nachfolgenden Wägeprozessen nur die Gewichtszunahme des Gesamtsystems über das Gewicht des die Vorflutflüssigkeit enthaltenden Systems hinaus bestimmt.

Zum nachfolgenden, eigentlichen Befüllschritt muss ein Ziel-Klemmventil, beispielsweise das in Figur 1 unterste Ziel-Klemmventil 26 geöffnet werden. Der Druck der Flüssigkeitssäule in der Hauptleitung 16 versucht in diesem Stadium, die Vorflutflüssigkeit aus der Hauptleitung 16 durch den Verbindungsschlauch 18a in den Einwegbeutel 20a zu drücken. Dies wird jedoch durch das Rückschlagventil 146 verhindert, welches sich bei dem durch das Absacken der Flüssigkeitssäule entstehenden Unterdruck in der Hauptleitung 16 schließt und ein Abfließen verhindert. Stattdessen kann frische Flüssigkeit aus dem Vorratsbehälter durch den Einlassschlauch 122 in den Verbindungsschlauch 18a und den Einwegbeutel 20a gepumpt werden. Die Gewichtszunahme wird von der elektronischen Waage registriert. Dadurch, dass sich der Füllstand in der Hauptleitung 16 aufgrund des Rückschlagventils 146 nicht ändert, entspricht die gemessene Gewichtszunahme exakt der Befüllmenge des Einwegbeutels 20a. Nach Erreichen des gewünschten Füllstandes, was beispielsweise durch Überwachung einer Anzeige der elektronischen Waage festgestellt werden kann, wird das Ziel-Klemmventil 26a geschlossen. Mit dem Öffnen des nachfolgenden Ziel-Klemmventils 26b wird der Befüllvorgang des Einwegbeutels 20b eingeleitet, der im Wesentlichen wie eben für den Einwegbeutel 20a beschrieben erfolgt.

Durch Wiederholung des Prozesses können nacheinander sämtliche Einwegbeutel 20a, b, c exakt befüllt werden.

Bevorzugt werden alle der Behälteranordnung 10 zugehörigen Komponenten, d.h. sämtliche in Figur 1 dargestellten Komponenten mit Ausnahme der zur Befüllvorrichtung gehörigen Ventile 24, 26a, b, c und Regalböden 28a, b, c, als vorkonfektionierte, vorsterilisierte Einheit geliefert. Bei der Konfektionierung der Einheit können Kundenwünsche bzgl. Schlauchlängen und -durchmesser, Anzahl von Abzweigungen, Beutelgrößen etc. exakt berücksichtigt werden. Nach der Konfektionierung wird die gesamte Einheit in eine keimdichte Folie verpackt, bevorzugt eingeschweißt, und durch Hitze oder Bestrahlung, z.B. durch Gammastrahlung, sterilisiert. Derartige Handelseinheiten sind lange haltbar, einfach und platzsparend lagerbar und können ohne Schwierigkeiten in passende Befüllvorrichtungen eingesetzt werden, ohne dass die Gefahr einer Kontamination bestünde.

Natürlich stellen die in der speziellen Beschreibung diskutierten und in der Figur dargestellten Ausführungsformen nur illustrative Ausführungsbeispiele der vorliegenden Erfindung dar. Dem Fachmann ist im Lichte der hiesigen Offenbarung ein breites Spektrum an Variationsmöglichkeiten an die Hand gegeben. Insbesondere kann die Behälteranordnung, anders als in Figur 1 dargestellt, nicht aus einzelnen Elementen sondern im Wesentlichen einstückig bzw. stoffschlüssig konstruiert werden.

### Bezugszeichenliste

- 10: Behälteranordnung
- 12: Einlassabschnitt
- 122: Einlassschlauch
- 124: Sterilverbinder
- 14: Gasauslassabschnitt
- 142: Auslassschlauch
- 143: Abschlussmodul
- 144: Sterilfilter
- 146: Rückschlagventil
- 147: Ventilkörper
- 148: Ventilsitz
- 16: Hauptleitung
- 162: Hauptleitungsschlauch
- 164: Schlauchverbinder
- 18a, b, c: Verbindungsschlauch
- 20a, b, c: Einwegbeutel
- 22a, b, c: Schlauchverbinder
- 24: Vorrats-Klemmventil
- 26a, b, c: Ziel-Klemmventil
- 28a, b, c: Regalboden
- 30a, b, c: Schlauchschelle

## Patentansprüche

1. Behälteranordnung, umfassend eine Mehrzahl von flexiblen Einwegbeuteln (20a, b, c), welche jeweils ein Einlasselement aufweisen, an das ein flexibler Verbindungsschlauch (18a, b, c) angeschlossen ist, wobei jeder Verbindungsschlauch (18a, b, c) von einer flexiblen, gemeinsamen Hauptleitung (16) die eingangsseitig einen gemeinsamen Einlassabschnitt (12) aufweist, abzweigt,
**dadurch gekennzeichnet,**
**dass** die Hauptleitung (16) ausgangsseitig einen gemeinsamen Gasauslassabschnitt (14) aufweist, in dem ein entgegen der Gasauslassrichtung schließendes Rückschlagventil (146) und ein gasdurchlässiger, flüssigkeitsdichter Sterilfilter (144) angeordnet sind.

2. Behälteranordnung nach Anspruch 1,
**dadurch gekennzeichnet,**
**dass** das Rückschlagventil (146) und der Sterilfilter (144) in einem gemeinsamen Gehäuse angeordnet sind.

3. Behälteranordnung nach einem der vorangehenden Ansprüche,
**dadurch gekennzeichnet,**
**dass** das Rückschlagventil (146) in Gasauslassrichtung hinter dem Sterilfilter (144) angeordnet und als Gasventil ausgebildet ist.

4. Behälteranordnung nach einem der Ansprüche 1 bis 2,
**dadurch gekennzeichnet,**
**dass** das Rückschlagventil (146) in Gasauslassrichtung vor dem Sterilfilter (144) angeordnet und als Flüssigkeitsventil ausgebildet ist.

5. Behälteranordnung nach Anspruch 4,
**dadurch gekennzeichnet,**
**dass** alle inneren Elemente (147, 148) des Rückschlagventils (146) aus Kunststoff gefertigt sind.

6. Behälteranordnung nach einem der vorangehenden Ansprüche,
**dadurch gekennzeichnet,**
**dass** die flexiblen Einwegbeutel (20a, b, c) evakuiert sind.

7. Behälteranordnung nach einem der vorangehenden Ansprüche,
**dadurch gekennzeichnet,**
**dass** sie von einer flexiblen, gasdichten Hülle umgeben ist und als Gesamtheit sterilisiert wurde.

8. Verfahren zum Befüllen von flexiblen Einwegbeuteln (20a, b, c) einer Behälteranordnung nach einem der vorangehenden Ansprüche, wobei die Einwegbeutel (20a, b, c) in ein auf einer elektronischen Waage positioniertes Trägergestell (28a, b, c) eingesetzt sind, jeder Verbindungsschlauch (18a, b, c) in ein dem jeweils angeschlossenen Einwegbeutel (20a, b, c) zugeordnetes, steuerbares Ziel-Klemmventil 26a, b, c) eingelegt ist und der Einlassabschnitt (12) mit einem Vorratsbehälter verbunden und in ein steuerbares Vorrats-Klemmventil (24) eingelegt ist, wobei alle Klemmventile (24; 26a, b, c) zunächst geschlossen sind,
umfassend die folgenden Schritte:
a) Öffnen des Vorrats-Klemmventils (29),
b) Entweichenlassen von in der Hauptleitung (12, 14, 16) enthaltenem Gas durch den Gasauslassabschnitt (14),
c) Tarieren der elektronischen Waage,
d) Öffnen eines ersten Ziel-Klemmventils (26a),
e) Befüllen des zugeordneten Einwegbeutels (20a) unter Überwachung einer Gewichtsanzeige der elektronischen Waage bis zum Erreichen eines vorgegebenen Füllgewichts,
f) Schließen des ersten Ziel-Klemmventils (26a),
g) jeweils Wiederholen der Schritte c bis f für alle weiteren Ziel-Klemmventile (26b, c) und zugeordneten Einweg-Beutel (20b, c).

9. Verfahren nach Anspruch 8,
**dadurch gekennzeichnet,**
**dass** der Gasauslassabschnitt (14) nahe der höchsten Stelle der Behälteranordnung (10) positioniert ist.

## Claims

1. Container arrangement, comprising a plurality of flexible disposable bags (20a, b, c), which each comprise an inlet element with which a flexible connecting hose (18a, b, c) is connected, wherein each connecting hose (18a, b, c) branches off from a flexible, common main duct (16) having a common inlet section (12) at the inlet side, **characterised in that** the main duct (16) has at the outlet side a common gas outlet section (14) in which are arranged a non-return valve (146), which closes against the gas outlet direction, and a gas-permeable, liquid-tight sterile filter (144).

2. Container arrangement according to claim 1, **characterised in that** the non-return valve (146) and the sterile filter (144) are arranged in a common housing.

3. Container arrangement according to any one of the preceding claims, **characterised in that** the non-return valve (146) is arranged behind the sterile filter (144) in gas outlet direction and is constructed as a gas valve.

4. Container arrangement according to one of claims 1 and 2, **characterised in that** the non-return valve (146) is arranged in front of the sterile filter (144) in gas outlet direction and is constructed as a liquid valve.

5. Container arrangement according to claim 4, **characterised in that** all inner elements (147, 148) of the non-return valve (146) are made of plastics material.

6. Container arrangement according to any one of the preceding claims, **characterised in that** the flexible disposable bags (20a, b, c) are evacuated.

7. Container arrangement according to any one of the preceding claims, **characterised in that** it is surrounded by a flexible, gas-tight casing and was sterilised as a whole.

8. Method of filling flexible disposable bags (20a, b, c) of a container arrangement according to any one of the preceding claims, wherein the disposable bags (20a, b, c) are inserted into a support structure (28a, b, c) positioned on electronic scales, each connecting hose (18a, b, c) is placed in a controllable target clamping valve (26a, b, c) associated with the respectively connected disposable bag (20a, b, c) and the inlet section (12) is connected with a storage container and placed in a controllable storage clamping valve (24), wherein all clamping valves (24; 26a, b, c) are initially closed, comprising the following steps:
a) opening the storage clamping valve (29),
b) allowing gas contained in the main duct (12, 14, 16) to escape via the gas outlet section (14),
c) taring the electronic scales,
d) opening a first target clamping valve (26a),
e) filling the associated disposable bag (20a) while monitoring a weight indication of the electronic scales until reaching a predetermined filling weight,
f) closing the first target clamping valve (26a) and
g) respectively repeating the steps c to f for all further target clamping valves (26b, c) and associated disposable bags (20b, c).

9. Method according to claim 8, **characterised in that** the gas outlet section (14) is positioned near the highest point of the container arrangement (10).

## Revendications

1. Ensemble de récipients, comprenant une pluralité de sachets (20a, b, c) à usage unique souples qui présentent, chacun, un élément d'entrée, auquel est raccordé un tuyau (18a, b, c) de liaison souple, chaque tuyau (18a, b, c) de liaison étant dérivé d'une conduite (16) souple principale commune, qui présente une section (12) d'entrée commune du côté de l'entrée,
**caractérisé en ce**
**que** la conduite (16) principale présente une section (14) de sortie de gaz commune du côté de la sortie, dans laquelle un clapet (146) anti-retour, fermant le sens opposé à la sortie de gaz, et un filtre (144) stérile, perméable aux gaz, étanche vis-à-vis des liquides, sont disposés.

2. Ensemble de récipients selon la revendication 1,
**caractérisé en ce**
**que** le clapet (146) anti-retour et le filtre (144) stérile sont disposés dans un boîtier commun.

3. Ensemble de récipients selon l'une des revendications précédentes,
**caractérisé en ce**
**que** le clapet (146) anti-retour est disposé derrière le filtre (144) stérile dans le sens de sortie du gaz et est conçu sous la forme d'une soupape à gaz.

4. Ensemble de récipients selon l'une des revendications 1 à 2,
**caractérisé en ce**
**que** le clapet (146) anti-retour est disposé devant le filtre (144) stérile dans le sens de sortie du gaz et est conçu sous la forme d'une soupape à liquides.

5. Ensemble de récipients selon l'une des revendications précédentes,
**caractérisé en ce**
**que** tous les éléments (147, 148) internes du clapet (146) anti-retour sont fabriqués en matière synthétique.

6. Ensemble de récipients selon l'une des revendications précédentes,
**caractérisé en ce**
**que** les sachets (20a, b, c) à usage unique souples sont évacués.

7. Ensemble de récipients selon l'une des revendications précédentes,
**caractérisé en ce**
**qu'**il est entouré d'une enveloppe souple, étanche aux gaz et qu'il a été stérilisé comme une entité globale.

8. Procédé destiné au remplissage de sachets (20a, b, c) à usage unique souples d'un ensemble de récipients selon l'une des revendications précédentes, les sachets (20a, b, c) à usage unique étant mis en oeuvre dans un bâti support (28a, b, c) positionné sur une balance électronique, chaque tuyau (18a, b, c) de liaison est introduit dans la soupape (26a, b, c) à serrage, pouvant être commandée, adjointe au sachet (20a, b, c) à usage unique respectivement raccordé, et la section (12) d'entrée est reliée avec un récipient de réserve et est mise en oeuvre dans une soupape (24) à serrage de la réserve pouvant être commandée, toutes les soupapes (24 ; 26a, b, c) étant tout d'abord fermées,
comprenant les étapes suivantes :
a) ouverture de la soupape (29) à serrage de la réserve,
b) échappement du gaz contenu dans la conduite (12, 14, 16) principale par la section (14) de sortie de gaz,
c) tarage de la balance électronique,
d) ouverture d'une première soupape (26a) à serrage cible,
e) remplissage du sachet (20a) à usage unique correspondant en tenant compte de l'indication de poids de la balance électronique jusqu'à ce que le poids de remplissage prédéterminé soit atteint,
f) fermeture de la première soupape (26a) à serrage cible,
g) répétition respective des étapes c à f pour toutes les autres soupapes (26b, c) à serrage cibles et les sachets (20b, c) à usage unique correspondants.

9. Procédé selon la revendication 8,
**caractérisé en ce**
**que** la section (14) de sortie de gaz est positionnée près de la position la plus haute de l'ensemble (10) de récipients.
